(19) Europäisches Patentamt
European Patent Office
Office européen des brevets

(11) Publication number: **0 258 749 B1**

# (12) EUROPEAN PATENT SPECIFICATION

(45) Date of publication of patent specification: **17.06.92** (51) Int. Cl.[5]: **A61L 17/00**

(21) Application number: **87112087.9**

(22) Date of filing: **20.08.87**

## (54) Surgical filament coating.

(30) Priority: **05.09.86 US 903799**
**05.09.86 US 903791**
**05.09.86 US 903798**

(43) Date of publication of application:
**09.03.88 Bulletin 88/10**

(45) Publication of the grant of the patent:
**17.06.92 Bulletin 92/25**

(84) Designated Contracting States:
**AT BE CH DE ES FR GB GR IT LI LU NL SE**

(56) References cited:
**EP-A- 0 170 966**

(73) Proprietor: **AMERICAN CYANAMID COMPANY**
**1937 West Main Street P.O. Box 60**
**Stamford Connecticut 06904-0060(US)**

(72) Inventor: **Lehmann, Leonard Theodore**
**45 Aunt Hack Road**
**Danbury Connecticut 06811(US)**
Inventor: **Wang, David Wei**
**800 Overbrook Drive**
**Vestal New York 13850(US)**
Inventor: **Casey, Donald James**
**9 Revere Place**
**Ridgefield Connecticut 06877(US)**
Inventor: **Rosati, Louis**
**182 Sunrise Hill Road**
**Norwalk Connecticut(US)**
Inventor: **Jarrett, Peter Kendrick**
**American Cyanamid Company 1937 West Main Street**
**Stamford Connecticut 06904(US)**

(74) Representative: **Wächtershäuser, Günter, Dr.**
**Tal 29**
**W-8000 München 2(DE)**

EP 0 258 749 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a surgical filament having a coating.

EP-A-170 966 discloses a salt of a $C_6$ or higher fatty acid, which in dry powder form is useful as a coating for a synthetic surgical filament.

According to the present invention a surgical filament is provided having a coating comprising a compound of the formula

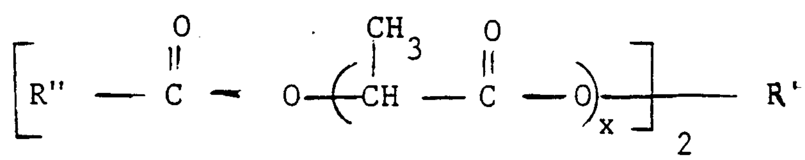

wherein x is 2 to 4, R' is selected from the group consisting of an alkaline-earth metal, aluminium and zinc ion or radical, and R" is an alkyl group having 12 to 22 carbon atoms in the backbone.

The surgical filament can be a bioabsorbable suture or ligature containing on the surface thereof a coating as defined above.

A coating composition of this invention comprises a compound of the formula:

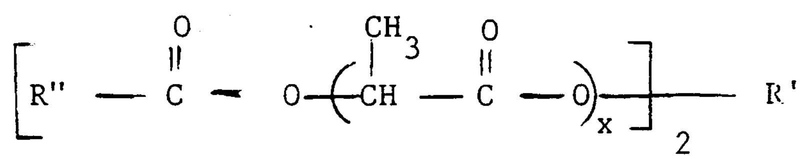

wherein x is 2 to 4, R' is selected from the group consisting of an alkaline-earth metal, aluminium and zinc ion or radical, and R" is an alkyl group having at 12 to 22 carbon atoms in the backbone. An ester of R" having from about 12 to 22 carbon atoms, and mixtures thereof, are preferred.

A compound of this formula can be prepared by methods known in the prior art. Illustrative of prepared compounds are magnesium, barium, aluminium or zinc palmitoyl lactylate; and calcium, magnesium, barium, aluminium or zinc oleoyl lactylate. Particularly preferred is a commercially available food grade of calcium stearoyl-2-lactylate.

Illustrative of solvents useful in preparing the calcium stearoyl-2-lactylate coating composition are the aromatic series of benzene, toluene, xylene and mesitylene, and the aliphatic solvents, 1,1,2-trichloroethane and chloroform.

A fatty acid ester coating composition of the above formula is conveniently applied to a suture by passing the suture through a container of the coating composition. Other conventional methods of applying coatings or finishes to continuous strands of fibers may also be used with substantially similar results, e.g. melt coating. Preferably, the application of the coating to the suture is regulated to provide from 1 to 5 percent dry coating by weight of the suture fiber.

The coating adheres well to the suture. Also, the coating is translucent so that the appearance of the coated suture is not significantly altered by the coating. Also, there is no appreciable dusting or flaking of the coating during suture tie-down.

In coating multifilament sutures with the fatty acid ester in accordance with the present invention, it is not necessary that every filament within the suture be individually or completely coated. Under most circumstances, the coating will penetrate at least partially into the interstices of the suture. What is critical is that the outer surface of the suture be well coated in order to reduce frictional forces during suture tie-down.

The amount of coating composition applied to the suture fiber, or the coating add-on, will vary depending upon the construction of the fiber. For example, the number of filaments and tightness of a braid or twist, and the nature of the coating material, e.g., its viscosity and consistency will vary the amount of coating applied to the fiber.

In general, the dry coating composition applied to a braid will constitute from 1 to 5 percent by weight of the coated suture fiber. However, it is to be understood that coating composition add-on may range from as little as 0.5 percent by weight to as much as 10 percent by weight or higher in some cases.

As a practical matter, and for reasons of economy and general performance, it is preferred to apply the

minimum amount of coating composition. This level of add-on can be determined without undue experimentation for any particular fiber-coating system, and is usually within the range of 2 to 4 dry weight percent, by weight of the coated suture fiber.

To achieve specific desired properties, the coating compositions of this invention can further be plasticized by various agents such as glyceryl triacetate, butyl citrate, ethyl benzoate, dibutyl phthalate. Various additives can also be included in the formulation to improve the performance of the lubricants such as calcium stearate or other salts of fatty acids, and bioabsorbable polyester and polyester-carbonate salts can be used. Also, water soluble lubricants such as a poly(alkylene oxide) can be included.

The coating composition can also include a poly($\beta$-hydroxy butyrate-co-$\beta$-hydroxyvalerate) and/or a poly($\beta$-hydroxybutyrate). A polymer of ($\beta$-hydroxybutyrate) can improve the knot run-down performance of a multifilament suture. Also, a polymer of ($\beta$-hydroxybutyrate) can be used as a binder, to hold the lubricant in place on the surface of a suture. Thus the lubricant can better resist displacement caused by friction during the knotting of the suture. Finally, tests indicate that poly($\beta$-hydroxybutyrate-co-$\beta$-hydroxy valerate), and mixtures thereof, with calcium stearoyl-2-lactylate performs well under both suture dry and wet conditions.

The improvement in suture properties imparted to a synthetic bioabsorbable suture may be determined semiquantitatively by comparing the hand of a coated and uncoated suture during the act of tying down a single throw knot. Such a comparison can be made on both a wet and dry suture since many suture coatings show different tie-down properties when tested wet or dry. It is to be understood that the term wet tie-down refers to the testing of a coated suture in an in-vivo or in-vitro simulated fluid, and the ten day tie-down refers to bench top testing of a coated suture without exposure to an in-viro or simulated fluid.

The present invention is further illustrated by example 3. Examples 1 and 2 being only "Reference examples", not part of the invention.

<u>EXAMPLE 1</u> (Reference)

Into a heated polymerization reactor was charged 45g of glycolide, 55g of trimethylene carbonate (TMC), 9.23 mg of $SnCl_2 \cdot 2H_2O$, and 97.21 of diethylene glycol (hereafter abbreviated as DEG). The reaction mixture was heated with stirring under dry nitrogen at 186° C for 75 minutes.

The resulting copolymer was discharged as a viscous melt which was cooled and ground to fine particles. The ground copolymer was dried under vacuum at 60° C overnight.

The copolymer had a composition of 47.7 weight percent trimethylene carbonate and 52.3 weight percent of glycolide. Less than 2.5 percent of monomeric trimethylene carbonate was present. The inherent viscosity was 0.98 dl/g.

<u>EXAMPLE 2</u> (Reference)

The copolymer of Example 1 was formulated as follows for evaluation as a coating for a braided absorbable suture:

<u>Coating Formulation 1</u> (Control)

6.0 grams of copolymer
9.0 grams of Xylene
80.0 grams of Methylene Chloride

<u>Coating Formulation 2</u>

6.0 grams of Calcium Stearoyl-2-Lactylate (Verv®)*
1.6 grams of copolymer
9.0 grams of Xylene
80.0 grams of Methylene Chloride

<u>Coating Formulation 3</u>

3.0 grams of Calcium Stearoyl-2-Lactylate (Verv®)*

*C.J. Patterson Co., Kansas City, MO. U.S. A.

3.0 grams of Copolymer
4.5 grams of Xylene
40.2 grams of Methylene Chloride

EXAMPLE 3

A commercially available poly($\beta$-hydroxybutyrateco- $\beta$-hydroxyvalerate) was obtained from ICI Ltd., U.K.and then placed into the following formulations:

Coating Formulation 4 (Control)

0.45 grams of polymer
0.70 grams of Xylene
6.00 grams of Methylene Chloride

Coating Formulation 5

6.0 grams of Calcium Stearoyl -2-Lactylate (Verv®)*
1.6 grams of polymer
9.0 grams of Xylene
80.0 grams of Methylene Chloride

Five 6.1 metres (20 Feet) lengths of size 2/0 polyglycolic acid braid was formed into five skeins. One skein was immersed in each of the formulations 1 to 5 above for 5 minutes. The skeins were then removed, allowed to drain, and dried for 1 hour. The dried skeins were then straightened and cut into suitable lengths.

Each length was then tied around a conventional tubular rubber tying board in the following manner. A single throw was made and then run down to the tubing to assess the resistance of the knot to rebound (the ability of the single throw to remain in position after the run down is complete). A square knot was then formed and run down to the tubing to assess the stick-slipping of the knot (which is also termed chatter) as it runs down and to assess the force required to initiate and sustain the run down.

The rating system used to evaluate these coatings was:

| | |
|---|---|
| Excellent | (a) No stick-slip during run down.<br>(b) Moderate force required which does not result in damage to the sleeve fibers of the suture.<br>(c) No rebound of the single throw. |
| Good | (a) No stick-slip during run down.<br>(b) Run-down force is a little high, but no damage is done to the sleeve fiber.<br>(c) Minor rebound of the single throw. |
| Fair | (a) Some stick-slip during run down.<br>(b) Run-down force is somewhat high and minor damage to the sleeve fiber is noted.<br>(c) Minor rebound of the single throw can occur. |
| Poor | (a) High stick-slip in run down.<br>(b) High damage or even breaking of the strand occurs.<br>(c) High rebound of the single throw occurs. |

The suture strands coated only with the glycolide/TMC copolymer of Formulation 1 were rated between Excellent and Good. The suture strands coated with the mixture of Verv® and the glycolide/TMC copolymer of Formulation 2 were rated Fair; the suture strands coated with the mixture of Formulation 3 were rated Excellent. The suture strands coated with Formulations 4 and 5 were both rated between Good and Excellent.

EXAMPLE 4

The commercially available poly($\beta$-hydroxybutyrate- co-$\beta$-hydroxyvalerate) of Example 3, herein "PHB-PHV", was formulated as follows:

Coating Formulation 6

4.0 grams PHB-PHV 70/30 polymer
4.0 grams of Calcium Stearoyl-2-Lactylate
16.84 grams Xylene (19.6 cc)
175.16 grams Methylene Chloride (133.1 cc)
All liquids are added to preweighed solids and the mixture is shaken for six hours at room temperature to get solubility.

A description of the coating method for the above suture and coating formulations is as follows. A commercially available coater (e.g. from the Bouligny Co., U.S.A.) is set to operate on a filament travelling at a speed of 15.25 metres (50 feet) per minute. The circulating air in the drying oven is adjusted to be 80 ° C.

There is only one pass of the filament through the capillary coating apparatus, and then through the drying oven. The coating pump is adjusted to give about 5 to 8 drops per minute at the capillary apparatus.

Using the above coating method, the percent pick up was about 3.5 to 3.6 percent based on the weight of the filament. It is to be understood that this amount of pickup can be increased or decreased by any person skilled in the art without undue experimentation by adjusting the above parameters. Preferably, the amount of pick up is increased by decreasing the amount of solvent in the formulation; and vice versa.

**Claims**

**1.** A surgical filament having a coating comprising a compound of the formula:

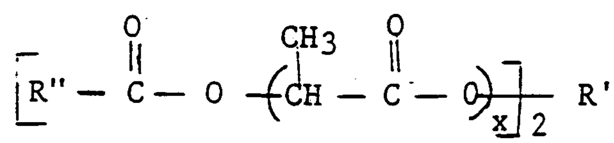

wherein x is 2 to 4, R' is selected from the group consisting of an alkaline-earth metal aluminium and zinc ion or radical, and R" is an alkyl group having 12 to 22 carbon atoms in the backbone.

**2.** A surgical filament having in combination a bioabsorbable suture or ligature and contained on the surface thereof a coating of claim 1.

**3.** A surgical filament of claim 2 wherein the suture or ligature is manufactured from a polymer containing at least one glycolic acid ester linkage, and the coating comprises a compound of the formula:

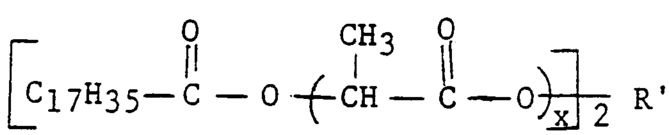

wherein x is 2 to 4 and R' is an alkaline-earth metal ion.

**4.** A surgical filament of claim 1 or 2 or 3 wherein x is 4 and/or R' is an ion selected from the group consisting of magnesium, calcium, barium, and zinc.

**5.** A surgical filament of claim 1 in combination with a lubricant additive of a stearoyl lactylate having the formula:

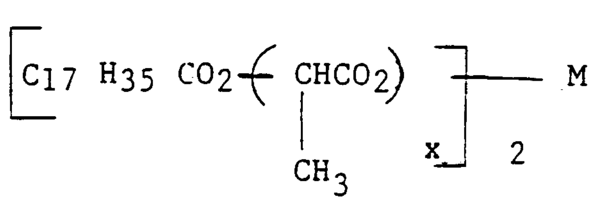

wherein X is two and M is an alkaline-earth metal ion.

## Revendications

**1.** Filament chirurgical ayant un revêtement comprenant un composé de formule :

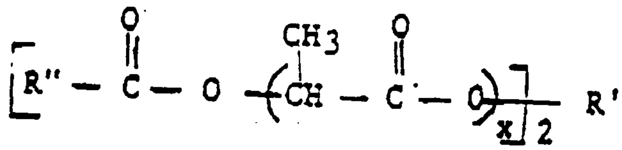

dans laquelle X est de 2 à 4, R' est choisi parmi le groupe consistant en un ion ou un radical d'aluminium et de zinc de métal alcalino-terreux, et R" est un groupe alkyle ayant 12 à 22 atomes de carbone dans son squelette.

**2.** Filament chirurgical comportant en combinaison une suture ou ligature bio-absorbable et à la surface de celle-ci un revêtement selon la revendication 1.

**3.** Filament chirurgical selon la revendication 2, dans lequel la suture ou ligature est fabriquée à partir d'un polymère contenant au moins une liaison ester d'acide glycolique, et le revêtement comprend un composé de formule :

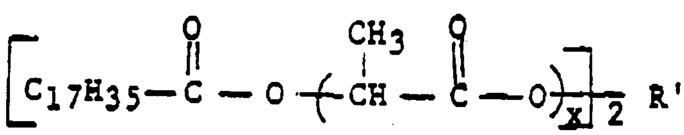

dans laquelle X est de 2 à 4 et R' est un ion de métal alcalino-terreux.

**4.** Filament chirurgical selon la revendication 1 ou 2 ou 3, dans lequel X est 4 et/ou R' est un ion choisi parmi le groupe consistant en magnésium, calcium, baryum, et zinc.

**5.** Filament chirurgical selon la revendication 1 en combinaison avec un additif lubrifiant constitué d'un lactylate de stéaryle ayant la formule :

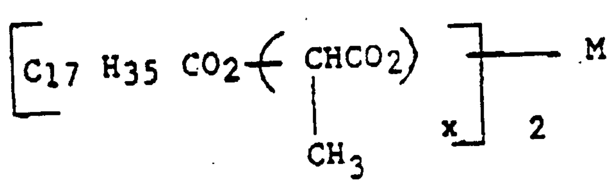

dans laquelle X est 2 et M est un ion de métal alcalino-terreux.

## Patentansprüche

**1.** Chirurgischer Faden mit einer Beschichtung, umfassend eine Verbindung der Formel

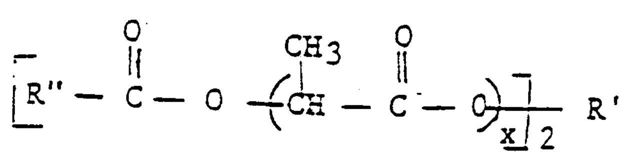

wobei x für 2 bis 4 steht, R' ausgewählt ist aus der Gruppe, bestehend aus einem Erdalkalimetall-, Aluminium- und Zinkion oder Radikal und R'' für eine Alkylgruppe steht, die 12 bis 22 Kohlenstoffatome im Rückgrat aufweist.

2. Chirurgischer Faden, der in Kombination ein bioabsorbierbares Naht- oder Ligaturmaterial aufweist und an der Oberfläche desselben eine Beschichtung gemäß Anspruch 1 enthält.

3. Chirurgischer Faden gemäß Anspruch 2, wobei das Naht- oder Ligaturmaterial hergestellt wurde aus einem Polymeren, enthaltend mindestens eine Glycolsäureesterverknüpfung, und wobei die Beschichtung eine Verbindung der folgenden Formel umfaßt:

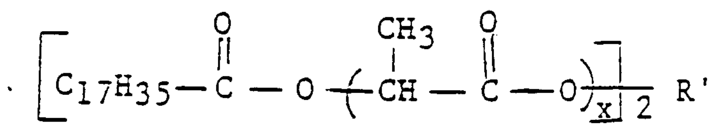

wobei x für 2 bis 4 steht und R' für ein Erdalkalimetallion steht.

4. Chirurgischer Faden gemäß Anspruch 1 oder 2 oder 3, wobei x für 4 steht und/oder R' ein Ion ist, ausgewählt aus der Gruppe, bestehend aus Magnesium, Calcium, Barium und Zink.

5. Chirurgischer Faden gemäß Anspruch 1 in Kombination mit einem Gleitmittelzusatz von einem Stearoyllactylat mit der Formel :

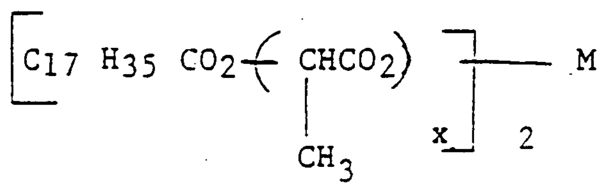

wobei x für 2 steht und M für ein Erdalkalimetallion steht.